# FASCICULE DE BREVET EUROPEEN

(11) **EP 2 763 786 B1**
(45) Date de publication et mention de la délivrance du brevet: **27.06.2018**
(21) Numéro de dépôt: 12769111.1
(22) Date de dépôt: 08.10.2012
(51) Int. Cl.: B01D 65/02, B01D 65/08, B01D 69/14

(54) **UTILISATION D'UN DISPOSITIF COMPRENANT UN MATÉRIAU COMPOSITE PRÉSENTANT DES NANOTUBES SOUMIS À UN CHAMP ÉLECTRIQUE**
VERWENDUNG EINER VORRICHTUNG MIT EINEM VERBUNDMATERIAL UND MIT EINEM ELEKTRISCHEN FELD UNTERWORFENEN NANORÖHRCHEN
USE OF A DEVICE COMPRISING A COMPOSITE MATERIAL AND HAVING NANOTUBES THAT ARE SUBJECTED TO AN ELECTRIC FIELD

(30) Priorité: 07.10.2011 FR 1159062
(43) Date de publication de la demande: 13.08.2014
(73) Titulaire: Commissariat à l'Énergie Atomique et aux Énergies Alternatives, 75015 Paris (FR)
(72) Inventeur: BOULANGER, Pascal, F-13510 Eguilles (FR); BROUZES, Alexandre, F-91190 Gif Sur Yvette (FR)
(74) Mandataire: Ahner, Philippe
(86) Numéro de dépôt international: PCT/EP2012/069851
(87) Numéro de publication internationale: WO 2013/050595

(56) Documents cités:
- WO-A1-2004/080578
- WO-A2-2008/062378
- US-B1- 7 229 556
- NAFICY S ET AL: "Modulated release of dexamethasone from chitosan-carbon nanotube films", SENSORS AND ACTUATORS A, ELSEVIER SEQUOIA S.A., LAUSANNE, CH, vol. 155, no. 1, 1 octobre 2009 (2009-10-01), pages 120-124, XP026688124, ISSN: 0924-4247, DOI: 10.1016/J.SNA.2009.07.021 [extrait le 2009-08-03]

## Description

### DOMAINE TECHNIQUE

La présente invention concerne le domaine des nanotechnologies et, plus particulièrement, le domaine des matériaux contenant des nano-objets tels que des nanotubes conducteurs ou semi-conducteurs et des dispositifs contenant ces matériaux.

Ainsi, la présente invention met en oeuvre un dispositif comprenant (1) au moins un matériau composé d'un ensemble de nanotubes conducteurs ou semi-conducteurs notamment alignés enrobés ou revêtus par une matrice et (2) des moyens permettant de polariser ce matériau i.e. de soumettre ce matériau à un champ électrique.

La présente invention porte sur l'utilisation d'un tel dispositif pour le décolmatage des membranes telles que des membranes de filtration.

### ÉTAT DE LA TECHNIQUE ANTÉRIEURE

La filtration membranaire à travers des membranes poreuses ou denses est connue depuis les premières expériences de l'Abbé Nolet, et utilisée industriellement notamment depuis les travaux de Sourirajan. Différents types de filtration sont actuellement utilisés en fonction de la taille des pores. Ainsi, on distingue :
- la microfiltration avec des membranes poreuses dont les pores sont de l'ordre du micromètre, utilisée pour la rétention des bactéries ;
- l'ultrafiltration avec des membranes poreuses constituées de mésopores, utilisée pour la rétention des macromolécules et virus ;
- la nanofiltration avec des membranes poreuses constituées de micropores, utilisée pour la potabilisation d'eau (rétention de micro-polluants) ;
- l'osmose inverse avec des membranes denses, utilisée en dessalement d'eau de mer.

En parallèle, les nano-objets suscitent actuellement un engouement particulier du fait de leurs propriétés originales et exacerbées par rapport aux matériaux classiques. En effet, les nano-objets présentent de nombreux intérêts aussi bien au niveau de leurs structures et de leurs propriétés physiques qu'au niveau de leurs applications potentielles notamment dans les membranes ou tout autre dispositif de séparation physique, les matériaux composites, les revêtements, les dispositifs thermiques, optiques ou électroniques, les électrodes, ainsi que les supports de catalyseurs et les dispositifs de stockage ou de conversion d'énergie chimique, lumineuse, électrique, mécanique, etc ....

Parmi les nano-objets, les nanotubes de carbone (NTC) découverts en 1991 font l'objet de nombreux travaux de recherche et d'applications. Ainsi, une mise en oeuvre particulière des NTC consiste à les imprégner dans une matrice permettant ainsi d'obtenir un matériau composite multifonctionnel, comme une membrane notamment utile en filtration, un revêtement avec des propriétés actives ou une électrode multifonctionnelle.

En effet, dans le domaine des applications membranaires, il a été prévu par simulation et démontré expérimentalement que, selon certaines conditions, la vitesse d'écoulement de l'eau dans le coeur de NTC pouvait être jusqu'à 1000 fois plus rapide que la vitesse prédite par les lois classiques de diffusion **[1].** On parle de « transport facilité » qui est défini comme le transport de molécules d'eau selon une organisation spatiale qui limite les frottements des molécules entre elles et avec les parois d'un pore et conduit à des vitesses d'écoulement supérieures aux lois de transport classiques. C'est aussi le cas mais à des degrés moindres pour les liquides neutres ou ioniques et les gaz. La réalisation de membranes à base de nanotubes alignés a été démontrée expérimentalement et ce, par plusieurs méthodes.

Que les membranes contiennent ou non des NTC et quelle que soit leur application, ces dernières sont sujettes au colmatage. En effet, les pores des membranes se bouchent sous l'effet combiné (1) du vieillissement du matériau, (2) de l'accumulation dans les pores de composés des solutions à filtrer et (3) de l'apparition dans les pores ou en surface de la membrane d'un film organique qui se développe et bouche les pores. Ce phénomène est connu sous le nom anglais de « bio-fouling », cet encrassement étant la conséquence de l'accumulation, de la croissance et de la prolifération d'un « biofilm » constitué de résidus d'espèces biologiques et de micro-organismes tels que des bactéries.

Pour éviter le colmatage des membranes, de nombreuses stratégies de prévention et de traitement sont utilisées mais elles reposent essentiellement sur :
- la prévention par traitement de surface avec des couches bactéricides de diverses natures (chimique ou nanoparticules). Il est à noter que les membranes à nanoparticules et/ou NTC sont intrinsèquement plus résistantes à la prolifération de biofilm du fait de leurs propriétés électriques (**[2]** et **[3]**) et de la structure des NTC en tapis de fakir hydrophobe.
- l'utilisation de composés chimiques qui vont détruire chimiquement les germes, les cristaux et retarder l'apparition des bouchages : c'est le nettoyage. Ce traitement est un traitement chimique essentiellement à base de chlore ou de produits anti-fouling qui eux-aussi vont avoir tendance, à terme, à se déposer sur les membranes. Il faut prévenir ces précipitations avec d'autres traitements chimiques. Ce type de traitement est le seul utilisable pour les membranes asymétriques et, en particulier, pour les membranes de dessalement d'eau de mer.
- l'utilisation de la technique de nettoyage inverse (ou, en anglais, « back-wash ») qui consiste à faire fonctionner la membrane en inversant son flux standard pour « décoller » les particules/organismes qui se seraient logés au sein de ses pores. Cette stratégie est notamment utilisée pour les membranes dites « symétriques » qui permettent le passage des solutés dans les deux sens.

On peut citer aussi des techniques faisant appel à des stratégies actives comme, par exemple, la mise en vibration de la membrane par des techniques piézo-électriques **[4]** ou par oxydation des produits colmatants **[5],** ou encore par génération de bulles de gaz directement à l'interface, jouant le rôle de « slug flow » connu pour son efficacité dans d'autres systèmes **[6].**

Une autre technique actuellement en cours de développement consiste à comprendre comment utiliser ce « colmata » ou biofilm afin qu'il se régule lui-même sans endommager la membrane. Cette stratégie s'applique surtout au cas du colmatage par des produits organiques et est basée sur la connaissance précise du mode de développement du biofilm pour le limiter.

Les NTC, lorsqu'ils sont soumis à des tensions, permettent d'exalter le phénomène dit d'émission de champ par effet de pointe, ce qui a pour conséquence d'augmenter le champ émis pour une tension donnée par rapport à un matériau non structuré. Les NTC individuels ont été massivement employés pour des études en émission de champ **[7].** En effet, leur forme géométrique cylindrique (facteur de forme > 100), couplée avec leurs propriétés de conduction électronique (transport balistique sur 100 nm⁻¹, densité de courant transportable > 10⁹ A/cm², mobilité électron-trou proche < 10⁵ cm²/V.s), ainsi qu'une grande stabilité chimique, thermique et mécanique en font un matériau idéal pour l'émission de champ.

De la même façon, de nombreuses études ont trait à la mise sous haute tension de NTC essentiellement individualisés. Celles-ci mentionnent, en plus de l'effet de champ (de pointe ou de parois), des comportements particuliers. En effet, si la tension appliquée au nanotube est trop forte, celui-ci peut soit se détruire par l'un de ses bouts, soit se casser au voisinage de son milieu. De plus, la mise sous tension des nanotubes est déjà connue pour avoir un effet bénéfique tant au niveau de leur synthèse (PECVD pour « Plasma Enhanced Chemical Vapor Déposition ») que sur la qualité de leur cristallinité.

Les formules théoriques ne sont pas simples et explicites mais, dans le cas d'une membrane de densité 10⁹ nanotubes/cm² avec des nanotubes de diamètre interne de 1 nm et de longueur 100 nm, l'amélioration de l'effet de champ en intensité par rapport à une électrode métallique classique peut être estimée à un facteur (i.e. intensité du champ magnétique émis par la surface à tension appliquée équivalente) > 500 pour un nanotube unique **[8].** Pour un tapis dont les nanotubes sont espacés de 100 nm, celui-ci est diminué d'un facteur 10 (soit > 50 au total).

Ce phénomène est mis à profit essentiellement pour la réalisation d'écrans (émission de champ pour émission d'électrons). Le comportement des nanotubes individualisés sous champ est également mis à profit pour les aligner (diélectrophorèse). Ils jouent aussi de manière individuelle, un effet d'amplification local.

L'utilisation de l'effet de champ émis par les nanotubes de carbone a également été proposée pour l'électroporation des cellules.

Pour rappel, l'électroporation de cellules est une technique connue depuis plusieurs dizaines d'années. Elle consiste à la mise sous champ électrique variable d'une solution contenant des cellules biologiques, des protéines voire même des organes (cas de l'électrotransfert pour la vectorisation de susbstances médicamenteuses ou d'ADN). En fonction de la tension appliquée, l'action du champ pulsé va causer des actions réversibles ou irréversibles aux constituants d'origine biologique qui y sont soumis tels que (i) ouverture de pores i.e. création de pores ou perméabilisation de la membrane plasmique ; (ii) éclatement de la membrane plasmique ; (iii) dénaturation des protéines et des biopolymères.

L'électroporation classique nécessite l'emploi de champs pulsés intenses de l'ordre de 10⁴ à 10⁵ V/cm, ce qui nécessite des électrodes spécifiques et des tensions d'alimentation importantes.

Le choix des différentes fonctions d'utilisation se fait sur le choix de la tension d'alimentation (intensité d'impulsions) et du cycle d'alimentation (nombre d'impulsions et espacement entre les impulsions). En effet, plus la tension est forte et les cycles longs, plus l'effet irréversible est fort. A faible tension, des cellules peuvent être électroporées sans être détruites et, à forte tension, les cellules éclatent et sont éliminées. De la même manière, plus les pulses sont nombreux et rapprochés, plus l'effet est fort. Il est facile de trouver, dans l'état de la technique, des protocoles et des valeurs d'intensité de champ électrique qui permettent de réaliser respectivement une inactivation ou une perméabilisation cellulaire par électroporation et ce, aussi bien pour des microorganismes que pour des cellules animales.

Yantzi et Yeow ont montré la possibilité d'utiliser des tapis de nanotubes seuls aléatoirement alignés, comme électrode de système d'électroporation avec succès **[9].** De même, l'utilisation des nanotubes comme vecteurs à l'électroporation de cellules à déjà été proposée pour des nanotubes en solution pour lesquels la mise en contact de la cellule se fait sur les parois du tubes et non sur la pointe **[10].**

De manière différente, Rojas-Chapana et al. **[11]** ainsi que les inventeurs de la demande internationale WO 2008/062378 [10] ont démontré que l'effet de champ émis par des nanotubes dispersés en solution peut être utilisé pour exacerber la tension inter-électrode. Certaines cellules ayant la propriété de venir se coller aux nanotubes, celles-ci seront alors soumises directement à un champ local (ré-émission par la pointe des nanotubes en solution) plus fort que le champ ambiant. Dans la référence **[11],** l'effet de champ axial qui correspond au champ dans l'axe du tube et qui est le plus fort est utilisé, alors que, dans la référence **[12],** est utilisé l'effet de champ radial qui est perpendiculaire à l'axe des tubes, là où se collent les cellules.

Le brevet US 7,229,556 propose des dispositifs et procédés pour la délivrance cutanée d'une composition, utilisant une membrane formée par une pluralité d'objets nanotubulaires creux, revêtus d'une matrice polymère continue et un réservoir en communication fluidique avec cette membrane. L'extrémité des objets nanotubulaires et notamment des nanotubes est fonctionnalisée par des groupements dans le but de contrôler le débit de la composition à travers la membrane. L'application d'une impulsion électrique permet sélectivement d'occulter ou d'exposer les nanopores fonctionnalisées par de tels groupements, lorsque ces derniers sont chargés.

La demande internationale WO 2004/080578 propose des procédés et dispositifs pour purifier des fluides en mettant en oeuvre un matériel nanotechnologique comprenant des nanotubes défectueux choisis parmi des nanotubes imprégnés, fonctionnalisés, dopés, chargés, revêtus et irradiés. Cette demande envisage que les nanotubes se trouvent dans un milieu solide pour former une nanomembrane et que cette nanomembrane soit soumise à des champs électromagnétiques pour la mettre en vibration et éjecter les contaminants de sa surface.

Les inventeurs se sont fixés pour but de proposer une membrane plus résistante au colmatage que les membranes de l'état de la technique mais aussi permettant des stratégies de décolmatage plus faciles.

### EXPOSÉ DE L'INVENTION

La présente invention permet de résoudre les problèmes techniques tels que précédemment définis et d'atteindre le but que se sont fixés les inventeurs. La présente invention concerne l'utilisation d'un dispositif selon la revendication 1.

Les travaux des inventeurs ont mis à profit les propriétés d'effet de champ émis par les nanotubes inclus dans un matériau composite du type nanotubes imprégnés par ou incorporés dans une matrice, et soumis à un champ électrique et notamment des matériaux présentant un tapis de nanotubes alignés avantageusement de forte densité. Ainsi, les nanotubes alignés peuvent présenter une densité, exprimée en nanotubes/cm², supérieure ou égale à 10⁵ et notamment supérieure ou égale à 10⁹. Une telle utilisation permet de limiter, de réduire et/ou d'empêcher le colmatage de membranes comprenant de tels matériaux.

Ainsi, la présente invention propose une alternative « électrique » au nettoyage des membranes à base de tapis de nanotubes alignés en utilisant le principe de l'électroporation pour la destruction en continu et en fonctionnement du biofilm aux premiers stades de sa création et/ou une fois ce dernier formé.

De façon remarquable, cet effet de champ peut également être mis en oeuvre pour électroporer des substrats. En effet, la réalisation de dispositifs d'électroporation pour les applications biologiques peut bénéficier des avantages de nanotubes alignés car ceux-ci présentent des propriétés d'émission de champ qui permettent de diminuer les tensions appliquées et d'en diminuer la taille rendant de fait le dispositif plus compact et plus solide grâce à l'imprégnation des nanotubes et cette technique compatible avec des systèmes intégrés du type « biochips » ou « lab-on-chips ». Le tapis de nanotubes alignés imprégné est alors utilisé comme électrode d'électroporation.

Fait intéressant, la présente invention s'applique non seulement aux NTC mais également à tout type de nanotubes semi-conducteurs ou conducteurs.

Plus particulièrement, il est décrit un dispositif comprenant :
i) un matériau composite comprenant (1) une pluralité de nanotubes conducteurs ou semi-conducteurs, et (2) une matrice disposée entre ces nanotubes ; et
ii) des moyens permettant de soumettre ledit matériau composite à un champ électrique.

Dans le cadre de la présente invention, on entend par « nanotube » une structure tubulaire et/ou cylindrique dont le diamètre interne varie entre 0,5 nm et 100 nm, notamment entre 0,5 nm et 50 nm et, plus spécifiquement, pour des applications de nanofiltration entre 0,5 nm et 10 nm. Le diamètre externe de telles structures varie entre 0,5 nm et 100 nm, notamment entre 0,5 nm et 50 nm et, plus spécifiquement, pour des applications de nanofiltration entre 0,5 nm et 10 nm.

Les nanotubes conducteurs ou semi-conducteurs mis en oeuvre dans le cadre de la présente invention peuvent être des nanotubes inorganiques, des nanotubes organiques ou un mélange de nanotubes inorganiques et de nanotubes organiques.

Ainsi, tout ou partie des nanotubes conducteurs ou semi-conducteurs mis en oeuvre peuvent être des nanotubes inorganiques. De tels nanotubes inorganiques sont avantageusement choisis dans le groupe constitué par des nanotubes d'oxyde de zinc (ZnO), des nanotubes de nitrure de gallium (GaN), de nitrure de Bore (BN), des nanotubes du bisulfure de tungstène (WS₂), des nanotubes de bisulfure de molybdène (MoS₂), des nanotubes de séléniure de tungstène (WSe₂), des nanotubes de dioxyde de titane (TiO₂) ou des nanotubes de trioxyde de molybdène (MoO₃) ou un de leurs mélanges.

Parmi les nanotubes utilisables dans le cadre de la présente invention, il convient de noter que certains comme les nanotubes de nitrure de Bore (BN) présentent des propriétés intrinsèques piézo-électriques.

De même, tout ou partie des nanotubes conducteurs ou semi-conducteurs mis en oeuvre peuvent être des nanotubes organiques. De tels nanotubes organiques sont avantageusement des nanotubes de carbone. Un nanotube de carbone est défini comme un enroulement concentrique d'une (ou de plusieurs) couche(s) de graphène (pavage d'hexagones de carbone). On parle :
- de nanotubes mono-feuillet, de nanotubes simple-parois ou de SWNT (pour « Single Wall NanoTube ») lorsqu'il s'agit d'une seule couche de graphène ;
- de nanotubes double-feuillets, de nanotubes double-parois ou de DWNT (pour « Double Wall NanoTube ») dans le cas de deux couches de graphène ;
- de nanotubes multi-feuillets, de nanotubes multi-parois ou de MWNT (pour « Multi Wall NanoTube ») dans le cas de plusieurs couches de graphène.

La présente invention s'applique à tout type de nanotubes de carbone et ce quel que soit leur procédé de préparation. Ainsi, les nanotubes de carbone mis en oeuvre dans le cadre de la présente invention peuvent être des nanotubes à une seule couche de graphène (SWNT), des nanotubes à deux couches de graphène (DWNT), des nanotubes à plusieurs couches de graphène (MWNT) ou un de leurs mélanges.

L'homme du métier connaît différents procédés permettant de préparer des nanotubes tels que précédemment définis. A titre d'exemples plus particuliers de procédés permettant de préparer des NTC, on peut citer les procédés physiques basés sur la sublimation du carbone tels que des méthodes d'arc électrique, d'ablation laser ou utilisant un four solaire et les procédés chimiques tels que le procédé CVD ou consistant à pyrolyser des sources carbonées sur des catalyseurs métalliques.

Les tapis de NTC alignés sont avantageusement obtenus par croissance simultanée et parallèle de nanotubes uniques, perpendiculairement à un substrat, dont ils sont solidaires. La technique plus particulièrement mise en oeuvre pour la croissance de telles structures ordonnées est la CVD et notamment la CVD avec pré-dépôt de catalyseur ou avec co-injection simultanée de catalyseur et de sources carbonées.

Les nanotubes mis en oeuvre dans le cadre de la présente invention peuvent présenter une quelconque chiralité et une quelconque longueur. Avantageusement, ces nanotubes présentent une longueur comprise entre 10 nm et 2 cm, notamment entre 20 nm et 1 mm, en particulier entre 50 nm et 100 µm et, tout particulièrement, entre 100 nm et 50 µm.

Dans le matériau composite mis en oeuvre dans la présente invention, les nanotubes présentent, les uns par rapport aux autres, une conformation alignée.

Dans le cas d'une telle conformation alignée, les nanotubes sont utilisés alignés les uns par rapport aux autres dans une matrice verticale pour « vertical array ». Dans cette conformation, ils sont généralement et substantiellement perpendiculaires à un support. En d'autres termes, les nanotubes sont alignés en parallèle les uns par rapport aux autres et, avantageusement, verticalement dans une matrice verticale. On parle de « tapis », de « forêts » ou de « réseaux » de nanotubes.

Une conformation alignée peut être obtenue dès la préparation des nanotubes ou une fois ces derniers préparés notamment par des techniques de filtration dans le coeur des nanotubes telles que décrites dans la demande internationale WO 2008/028155 [12] ou des techniques par un flux transverse à l'axe des tubes telles que décrites dans la demande de brevet US 2004/0173506 [13] et la demande internationale WO 2009/141528 [14]**.**

La densité de nanotubes dans le matériau selon la présente invention peut être variable. Cette dernière est avantageusement comprise entre 10⁴ et 10¹³ nanotubes/cm² de matériau. La notion de densité de nanotubes est bien connue de l'homme du métier et correspond au nombre de nanotubes alignés par unité de surface. Cette densité se mesure classiquement par décompte des images de microscopie électronique des nanotubes (en absolu).

Densité, alignement et tortuosité des nanotubes sont aussi des paramètres accessibles par mesures aux rayons X et notamment comme décrit dans l'article de Pichot et al., 2004 **[15]** pour des MWNT.

Dans le matériau composite mis en oeuvre dans la présente invention, la matrice qui est disposée entre les nanotubes i.e. la matrice de remplissage peut être choisie dans le groupe constitué par une matrice céramique, une matrice polymère, une matrice métallique, une matrice issue de la biomasse ou une matrice issue de dérivés cellulosiques et leurs mélanges.

Par « matrice céramique », on entend plus particulièrement une matrice dont le matériau la constituant est choisi dans le groupe constitué par un matériau sol-gel, le nitrure de silicium, le nitrure d'aluminium, le nitrure de titane, le carbure d'aluminium, le carbure de titane, le carbure de silicium, l'oxyde de silicium, le dioxyde de silicium, l'oxyde de magnésium, l'oxyde de cérium, l'alumine, l'oxyde de titane, l'oxyde de bismuth, l'oxyde de beryllium, l'hydroxyapatite ou un de leurs mélanges.

La matrice polymère mise en oeuvre dans le cadre de la présente invention peut être constituée d'un (ou plusieurs) polymère(s) thermoplastique(s), d'un (ou plusieurs) polymère(s) thermodurcissable(s), d'un (ou plusieurs) polymère(s) vitreux ou d'un de leurs mélanges. Par « matrice polymère », on entend plus particulièrement une matrice dont le matériau la constituant est choisi dans le groupe constitué par un polyamide, un polyimide, un parylène, un polycarbonate, un polydiméthylsiloxane, une polyoléfine, une polysulfone, un polyéthersulfone, un polyétheréthercétone (PEEK) et ses dérivés, un polypropylène (PP), un polyfluorure de vinylidène (PVDF), un polyvinyl pyrrolidone (PVP), un acétate de cellulose (AC), une résine acrylique, un polystyrène (PS), un polyméthylméthacrylate (PMMA), un polyméthacrylate (PMA), une résine époxy, un polyester, un acétylnitrile-butadiène-styrène ou un de leurs mélanges.

De plus, la matrice de remplissage et, notamment lorsqu'il s'agit d'une matrice polymère, le matériau la constituant peuvent être susbtitués par au moins une fonction réactive. Par « fonction réactive », on entend, dans le cadre de la présente invention, une fonction choisie parmi une fonction carboxyle (susceptible de réagir avec une fonction amine ou alcool), un groupe aryle (tel que le pyrène, le naphtalène ou les polyaromatiques), une entité radicalaire, une fonction hydroxyle ou une fonction alcool (susceptible de réagir avec une fonction carboxyle ou isocyanate), une fonction amine (susceptible de réagir avec une fonction ester), une fonction ester (susceptible de réagir avec une fonction amine), une fonction aldéhyde (susceptible de réagir avec une fonction hydrazide), une fonction hydrazide (susceptible de réagir avec une fonction aldéhyde), une fonction cétone (susceptible de réagir avec deux fonctions alcool en vue d'une acétalysation), une fonction époxy (susceptible de réagir avec une fonction amine), une fonction isocyanate (susceptible de réagir avec une fonction hydroxyle), une fonction maléimide (susceptible de réagir avec une fonction thiol, une fonction amine ou une fonction diène), une fonction diène (susceptible de réagir avec une fonction maléimide) et une fonction thiol (susceptible de réagir avec une fonction maléimide ou une autre fonction thiol).

La matrice de remplissage peut être non poreuse ou poreuse. En effet, une matrice poreuse, du type polyamide, polysulfone, polyestersulfone, PP, PVDF, PVP ou acétate de cellulose, peut être particulièrement intéressante lorsque le matériau composite selon l'invention est utilisé pour des applications de dessalement d'eau de mer ou d'eau saumâtre.

En variante, lorsque le matériau composite mis en oeuvre dans la présente invention est utilisé comme une membrane de filtration, la matrice de remplissage peut être une structure imperméable à l'eau et ce, pour favoriser le passage du liquide à filtrer au sein des nanotubes.

En variante encore, lorsque le matériau composite mis en oeuvre dans la présente invention est utilisé comme une électrode, la matrice de remplissage est une matrice conductrice ou isolante et, avantageusement une matrice isolante.

Ainsi, le matériau composite mis en oeuvre dans le cadre de la présente invention peut être une électrode ou une membrane. Il peut également s'agir d'un revêtement hydrophobe à base de nanotubes de carbone et notamment un revêtement anti-fouling marin. Pour rappel, un revêtement hydrophobe à base de nanotubes de carbone présente des propriétés de super-fluidité, lorsqu'un liquide, par exemple de l'eau, glisse sur sa surface.

Toute technique connue de l'homme du métier pour disposer une matrice entre des nanotubes est utilisable pour préparer le matériau composite utile dans la présente invention. Cette étape permet de combler l'espace inter-nanotubes résiduel avec une matrice telle que précédemment définie.

Cette technique peut être un dépôt chimique en phase vapeur (CVD), un dépôt de couche atomique (ALD), un dépôt par centrifugation connu sous l'appellation anglaise de « spin coating » ; une imprégnation assistée ou non par pression ; une photo-imprégnation ; etc ....

De même, la matrice de remplissage peut être disposée dans tout l'espace entre les nanotubes ou au contraire au niveau de certaines parties de cet espace, laissant d'autres parties d'espace libre entre les nanotubes. Au contraire, la matrice de remplissage peut être disposée de sorte que les nanotubes soient enterrés dans la matrice.

Le matériau composite mis en oeuvre dans le cadre de la présente invention se présente sous forme d'une membrane isoporeuse dont les nanopores correspondent aux pores des nanotubes et éventuellement aux pores de la matrice de remplissage. Dans un tel matériau, les nanotubes sont dits « traversants ». les nanotubes débouchent au moins d'un côté du matériau composite et notamment au niveau de deux côtés opposés du matériau composite. Dans cette configuration, les nanotubes sont aussi dits « passants » dans la mesure où tout ou partie des nanotubes va participer au transport des espèces lors de la filtration via la membrane (i.e. le matériau composite).

Dans une forme de mise en oeuvre toute particulière du matériau composite mis en oeuvre dans la présente invention, les nanotubes sont covalemment greffés (ou fonctionnalisés ou dérivatisés) avec au moins un polymère organique, préalablement à leur incorporation par la matrice de remplissage. En d'autres termes, au moins un polymère organique est covalemment greffé sur chaque nanotube.

En effet, les travaux des inventeurs ont permis de montrer qu'ajouter une couche d'accroche liée de façon covalente aux nanotubes, notamment alignés, préalablement à leur imprégnation par (ou leur incorporation dans) une matrice, notamment une matrice polymère permet d'améliorer l'interface entre nanotubes et matrice.

Tout d'abord, le fait que la couche d'accroche soit greffée de manière covalente sur la surface des nanotubes renforce la liaison avec les nanotubes et permet donc une meilleure adhérence de la matrice de remplissage sur les nanotubes. En effet, lors de l'étape d'imprégnation, la matrice de remplissage interagit fortement avec la couche précédemment greffée. Le réseau interpénétré ainsi obtenu améliore la qualité de l'interface nanotubes/matrice et renforce les propriétés mécaniques du matériau.

De plus, cette interpénétration peut s'accompagner d'une physisorption mais aussi d'une chimisorption rendant les interactions entre la couche d'accroche et la matrice de remplissage plus fortes. Dans le cadre de la chimisorption, peuvent exister des liaisons ioniques ou des liaisons covalentes impliquant un atome de la couche d'accroche et un atome du matériau constituant la matrice de remplissage.

Ainsi, une meilleure interface entre la sous-couche et la matrice se traduit par une meilleure tenue mécanique et une meilleure imperméabilité de l'espace inter-nanotubes. Dans le cas des matrices de remplissage de type polymère, l'interpénétration plus forte des chaînes de polymères de la sous-couche avec les chaînes du matériau constituant la matrice de remplissage favorise une continuité entre ces chaînes.

L'amélioration de l'interface nanotubes/matrice réduit également les chemins de diffusion des liquides et/ou des gaz dans le matériau et permet à la fois une meilleure imperméabilité de l'espace inter-tube quand cela est recherché, et aussi une meilleure sélectivité en évitant que les espèces que l'on souhaite trier ne se remélangent via ces chemins de diffusion secondaires.

L'emploi d'une couche d'accroche et de fonctionnalisation adaptée permet une meilleure compatibilité avec le matériau de la matrice de remplissage et le remplissage par cette matrice de l'espace inter-nanotubes peut être partiel ou total. Ainsi, le remplissage partiel de l'espace inter-tubes peut être utilisé pour changer l'hydrophobicité des nanotubes et contrôler leur mouillabilité mais aussi pour créer une couche de sites d'adsorption pour transformer les nanotubes et notamment le tapis qu'ils forment en capteur, en électrode ou en filtre sélectif.

Par « polymère organique », on entend un polymère dont la chaîne principale comprend principalement des atomes de carbone mais peut aussi comprendre des hétéroatomes tels que des atomes d'oxygène et des atomes d'azote.

Ce polymère organique est avantageusement greffé, de façon covalente, sur la partie externe latérale des nanotubes. Ce greffage peut être localisé sur des zones limitées et définies de ces surfaces.

Chaque nanotube peut comprendre au moins un, au moins deux, au moins cinq, au moins dix, au moins 20 ou au moins 100 polymères organiques greffés, chaque polymère organique greffé sur un même nanotube pouvant présenter une séquence en unités identique avec, ou différent de l'autre (ou des autres) polymère(s) greffé(s). De même, les polymères organiques greffés sur des nanotubes différents peuvent présenter une séquence en unités identique pour tous les nanotubes ou différente.

Le polymère organique mis en oeuvre dans le cadre de la présente invention comprend :
- au moins une unité dérivée d'un sel d'aryle clivable, et/ou
- au moins une unité dérivée d'un monomère présentant au moins une liaison de type éthylénique, et/ou
- au moins une unité dérivée d'un monomère présentant au moins deux fonctions carboxylique, et/ou
- au moins une unité dérivée d'un monomère présentant aux moins deux fonctions amine et/ou
- au moins une unité dérivée d'un monomère présentant une fonction carboxylique et une fonction amine. Le polymère organique mis en oeuvre dans le cadre de la présente invention est avantageusement constituée de motifs répétitifs correspondant à de telles unités.

La couche d'accroche que forment les polymères organiques greffés sur les nanotubes peut contenir un autre matériau nanoscopique, notamment tel que des nanoparticules métalliques (Cu, Ar, Ag, Co, Si, Au, Ti, Pd, Cr, Al ou Pt) ou d'oxydes métalliques (TiO₂, TiO, etc ...).

Avantageusement, le polymère organique mis en oeuvre dans le cadre de la présente invention est substitué par au moins une fonction réactive telle que précédemment définie.

Par « sel d'aryle clivable », on entend, dans le cadre de la présente invention, un sel d'aryle clivable choisi dans le groupe constitué par les sels d'aryle diazonium, les sels d'aryle d'ammonium, les sels d'aryle phosphonium, les sels d'aryle iodonium et les sels d'aryle sulfonium. Plus particulièrement, un sel d'aryle clivable utilisable dans le cadre de la présente invention est de formule (I) suivante :

R-N₂⁺, A⁻ (I)

dans laquelle :
- A représente un anion monovalent, notamment choisi parmi les anions inorganiques tels que les halogénures comme I⁻, Br⁻ et Cl⁻, les halogénoborates tels que le tétrafluoroborate, les perchlorates et les sulfonates et les anions organiques tels que les alcoolates et les carboxylates et
- R représente un groupe aryle, correspondant à une structure carbonée aromatique ou hétéroaromatique, éventuellement mono- ou polysubstituée, constituée d'un ou plusieurs cycles aromatiques ou hétéroaromatiques comportant chacun de 3 à 8 atomes, le ou les hétéroatomes pouvant être N, O, P ou S. Le ou les substituants peuvent contenir un ou plusieurs hétéroatomes, tels que N, O, F, Cl, P, Si, Br ou S ainsi que des groupes alkyles en C₁ à C₆ ou des groupes thioalkyles en C₄ à C₁₂ notamment.

Par « monomère présentant au moins une liaison de type éthylénique », on entend avantageusement un monomère présentant une insaturation vinylique, une insaturation allylique et/ou une insaturation acrylique.

De tels monomères sont choisis parmi les monomères de formule (II) suivante : 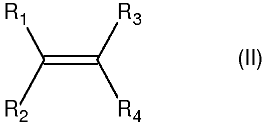 dans laquelle les groupes R₁ à R₄, identiques ou différents, représentent un atome monovalent non métallique tel qu'un atome d'halogène, un atome d'hydrogène, un groupe chimique saturé ou insaturé, tel qu'un groupe alkyle, aryle, un groupe -COOR₅ dans lequel R₅ représente un atome d'hydrogène ou un groupe alkyle en C₁-C₁₂ et de préférence en C₁-C₆, un nitrile, un carbonyle, une amine ou un amide. Avantageusement, les monomères présentant au moins une liaison éthylénique mis en oeuvre dans le cadre de la présente invention sont substitués par au moins une fonction réactive telle que précédemment définie.

Le procédé de préparation du matériau composite particulier tel que précédemment défini (i.e. nanotubes greffés + matrice de remplissage) présent dans le dispositif mis en oeuvre dans l'invention comprend les étapes successives consistant à :
a) greffer, sur la pluralité de nanotubes, au moins un polymère organique,
b) disposer entre les nanotubes obtenus suite à l'étape (a) une matrice telle que précédemment définie. L'étape (b) du procédé a déjà été explicitée.

Lors de l'étape (a) du procédé de préparation d'un matériau composite, toute technique permettant le greffage d'un polymère organique est utilisable. Cette dernière est avantageusement choisie parmi
- une fonctionnalisation des nanotubes suivie d'un couplage avec un polymère organique **[16] ;**
- un greffage chimique radicalaire basé sur le procédé décrit dans la demande internationale WO 2008/078052 [17] et dans l'article de Mévellec et al. 2007 **[18] ;**
- un électrogreffage, des informations complémentaires sur l'électrogreffage sur des nanotubes de carbone pouvant être obtenues dans l'article de Tessier et al., 2008 **[19] ;**
- un photogreffage ;
- un greffage par polymérisation radicalaire par transfert d'atomes ou ATRP (pour « Atom Transfer Radical Polymerization ») ;
- un greffage par polymérisation radicalaire contrôlée par le nitroxyde ou NMRP (pour « Nitroxide Mediated Radical Polymerization ») ;
- un greffage par polymérisation radicalaire par addition fragmentation tels que les procédés RAFT (pour « Reversible Addition Fragmentation chain Transfer ») ou MADIX (pour « MAcromolecular Design via Interchange of Xanthane ») ;
- un greffage en phase vapeur ou
- un greffage activé par les micro-ondes.

De plus amples informations sur les autres techniques de greffage susceptibles d'être utilisées lors de l'étape (a) du procédé pour préparer le matériau composite mis en oeuvre dans le cadre de la présente invention peuvent être obtenues dans l'article de Fan et al., 2007 **[20]** et dans les articles cités dans ce dernier.

Il convient de remarquer que les méthodes utilisées pour préparer la couche d'accroche ne perturbent pas l'alignement des nanotubes. Aussi, il est possible d'avoir un matériau présentant un tapis dense de nanotubes alignés, avec de l'ordre de 10⁵ à 10¹³ nanotubes/cm² et notamment de l'ordre de 10⁹ à 10¹¹ nanotubes/cm². Avantageusement, le désalignement maximum obtenu, suite au greffage covalent des polymères organiques, pour un tapis dense de nanotubes est de 10 degrés et la tortuosité maximale de 3%, et, en particulier, un désalignement de 5% pour une tortuosité de 1%.

Comme précédemment expliqué, l'utilisation combinée d'une couche d'accroche et d'une matrice de remplissage permet d'obtenir un matériau composite beaucoup plus solide qu'un tapis seul de nanotubes. Les nanotubes alignés dans le matériau composite présent dans le dispositif mis en oeuvre dans l'invention sont maintenus mécaniquement, ce qui permet d'obtenir une membrane ou une électrode beaucoup plus solide que les membranes et électrodes de l'état de la technique.

En effet, le matériau composite présent dans le dispositif mis en oeuvre dans l'invention, avec ou sans couche d'accroche, peut se présenter sous forme d'une membrane ou d'une électrode. Lorsque le matériau composite est utilisé comme une électrode, il peut être disposé sur ou supporté par un élément tel qu'un élément en silicium. Toutefois, le caractère autosupporté du matériau composite, notamment lorsqu'il présente une couche d'accroche, rend un tel support optionnel et non obligatoire comme dans certaines électrodes de l'art antérieur.

Pour des applications en tant que membrane, le matériau composite mis en oeuvre dans le cadre de la présente invention présente une matrice de remplissage avantageusement poreuse ou non poreuse, comme précédemment expliqué.

Pour des applications en tant qu'électrode, le matériau composite mis en oeuvre dans le cadre de la présente invention présente une matrice de remplissage conductrice ou isolante.

De plus, quelle que soit la matrice, il existe une conduction électrique dans l'axe des nanotubes mais aussi perpendiculairement à cet axe. En effet, il existe des contacts parasites qui diminuent le champ mais qui permettent une mise sous tension facilitée. De fait, il n'est plus nécessaire de contacter toute la base de l'électrode ou de la membrane. Ainsi, à titre d'exemples, pour un nanotube de carbone dont la longueur est de 100 µm, la résistance R dans l'axe du nanotube est comprise entre 0,1 et 100 ohms, pour un tapis de nanotubes de 1 cm de côté, la résistance transverse est comprise entre 10 et 500 ohms.

Que le matériau composite soit utilisé, dans le cadre de la présente invention, comme une membrane ou une électrode, il convient de noter que l'utilisation d'une matrice de remplissage isolante est avantageuse. En effet, l'emploi d'une matrice de remplissage isolante permet d'augmenter l'effet de champ des nanotubes et notamment du tapis de nanotubes en diminuant l'effet d'écrantage des nanotubes et notamment du tapis. On peut estimer que l'on gagne ainsi au moins un facteur 10 (sur l'utilisation d'un tel effet par rapport à un tapis simple).

De même, que le matériau composite soit utilisé comme une membrane ou une électrode, il peut se présenter sous différentes formes telles qu'une forme plane, nanostructurée ou spiralée.

Le dispositif mis en oeuvre dans le cadre de l'invention comprend en plus du matériau composite tel que précédemment défini des moyens permettant de soumettre à un champ électrique ce matériau et ainsi le polariser moyennant quoi l'effet de champ est généré.

Tout moyen permettant de soumettre à un champ électrique un matériau est utilisable dans le cadre de la présente invention. Avantageusement, les moyens mis en oeuvre dans le cadre de la présente invention sont choisis parmi des contacts électriques, une ou plusieurs électrodes et une source de tension.

Plus particulièrement, les moyens mis en oeuvre dans le cadre de la présente invention sont au moins une électrode et au moins une source de tension électriquement reliée, d'une part, au matériau composite tel que précédemment défini et, d'autre part, à ladite au moins une électrode. Ladite au moins une électrode est également désignée, dans la présente invention, sous le terme de « contre-électrode ». Il convient de noter que le matériau composite selon la présente invention et la contre-électrode doivent être isolées électriquement.

Cette électrode peut être en un quelconque matériau communément utilisé pour une électrode. A titre d'exemples non limitatifs, on peut citer une électrode comprenant une (ou plusieurs) couche(s) en un matériau métallique choisi parmi le cuivre, l'argent, le platine, le cobalt, l'or, le titane, le palladium, le chrome, l'aluminium et leurs alliages. Avantageusement, l'électrode mise en oeuvre peut être revêtue d'une couche isolante notamment en oxyde. En variante, cette électrode peut se présenter sous forme d'un fil en un matériau précité.

Dans une forme de mise en oeuvre particulière, cette électrode peut être en un matériau composite tel que précédemment défini.

De plus, le dispositif mis en oeuvre dans la présente invention peut comprendre au moins deux électrodes en plus du matériau composite.

Tout générateur de tension électrique connu de l'homme du métier est utilisable, dans le cadre de la présente invention, en tant que source de tension. La tension générée par la source de tension mise en oeuvre dans le dispositif selon l'invention peut être pulsée alternative ou continue. Ainsi, le champ électrique auquel est soumis le matériau composite du dispositif mis en oeuvre dans l'invention peut être continu ou variable.

Avantageusement, pour appliquer la stratégie d'électrolyse (oxydation des cellules et bullage), la tension est continue.

Il convient de noter que les valeurs de toutes les tensions sont données dans la présente invention sous forme de valeurs absolues et qu'elles doivent se comprendre comme pouvant être positives ou négatives. Ainsi, lorsqu'une tension pulsée est appliquée, la tension appliquée à chaque impulsion peut être positive ou négative et les tensions appliquées pour deux impulsions consécutives peuvent être de même signe ou de signe contraire. Il peut aussi s'agir d'une impulsion de fréquence variable (wobulation) alternative.

Dans un milieu liquide, en particulier en présence d'eau, l'utilisation de l'effet de pointe peut conduire à exalter le mécanisme de dissociation des molécules d'eau en molécules de di-hydrogène et de di-oxygène, plus connu sous le nom « d'électrolyse de l'eau ». Deux des effets de l'électrolyse de l'eau sous l'action d'un champ électrique sont :
- la possibilité d'oxyder des molécules et ainsi de les dégrader ;
- la possibilité de créer des bulles au voisinage de la surface du matériau composite, tel qu'une membrane, une électrode ou un revêtement, qui pourrait permettre de décoller le biofilm qui s'y serait accroché.

L'utilisation de ces deux phénomènes peut aussi être mise à profit dans le cadre de la présente invention en complément des techniques d'électroporation car ils nécessitent l'utilisation d'une tension continue et non variable. Le phénomène d'électrolyse de l'eau peut être facilement mis en évidence. En effet, en plaçant le matériau composite selon l'invention dans de l'eau en présence d'une contre-électrode du type fil et en appliquant une tension notamment de 5 V, l'apparition de bulles à la surface du matériau composite révèle cette électrolyse.

En variante, tout ou partie du dispositif selon l'invention peut être disposé sur un support tel qu'une biopuce, un puits d'une plaque multi-puits utilisé en biologie ou dans les biotechnologies, un récipient ou un container utile en agroalimentaire pour la conservation ou le traitement de denrées alimentaires comme du lait ou des jus de fruits. Avantageusement, le matériau composite et la contre-électrode sont disposés sur un tel support.

La présente invention concerne l'utilisation d'un dispositif pour désencrasser la surface et/ou les pores du matériau composite tel que précédemment défini.

Par « désencrasser », on entend, dans la présente invention, pour l'empêchement, la réduction et/ou l'élimination de l'encrassement.

Ainsi, la présente invention concerne un procédé pour empêcher, réduire et/ou éliminer l'encrassement d'un matériau composite et, plus particulièrement, l'encrassement à la surface, au niveau des pores et dans les pores (i.e. à l'intérieur des nanotubes) du matériau composite tel que précédemment défini, comprenant une étape consistant à soumettre ledit matériau composite à un champ électrique. Un tel procédé met en oeuvre le dispositif tel que précédemment défini.

Un tel procédé est applicable que le matériau composite se présente sous forme d'une membrane, d'une électrode ou d'un revêtement hydrophobe. Ce procédé concerne, avantageusement, l'encrassement des membranes notamment utilisées en filtration.

Dans le cadre de la présente invention, par « procédé pour empêcher l'encrassement », on entend un procédé préventif, appliqué au matériau composite et notamment à une membrane poreuse à base de nanotubes selon l'invention qui prévient tout début de germination minérale ou organique. De fait, le matériau ou la membrane résiste à l'encrassement. Dans ce cas, le champ électrique est généré préalablement à toute utilisation du matériau ou de la membrane et ce, pour protéger ces derniers de tout encrassement, en surface, au niveau des pores et dans les pores (i.e. à l'intérieur des nanotubes).

Les valeurs dans les exemples et variantes donnés ci-après s'entendent pour un matériau composite comprenant des nanotubes de carbone avec
- une densité de NTC variable et notamment comprise entre 10⁴ et 10¹³ nanotubes/cm² de matériau ;
- des NTCs avec des diamètres externes compris entre 2 et 500 nm, avantageusement entre 5 et 100 nm et plus particulièrement entre 5 et 80 nm ;
- des NTCs avec des diamètres internes compris entre 0,5 et 100 nm, avantageusement entre 1 et 30 nm et plus particulièrement entre 2 et 20 nm ;
- la longueur des NTCs correspond à l'épaisseur du matériau et est comprise entre 1 µm et 1 mm, notamment entre 2 et 800 µm et, en particulier, entre 3 et 100 µm.

Dans une 1^{ère} variante et à titre d'exemple, le procédé préventif selon l'invention peut être mis en oeuvre à l'aide d'un champ électrique constant auquel un matériau composite comprenant des nanotubes de carbone, est soumis en appliquant une tension constante, comprise entre 0,1 et 100 V et, notamment entre 1 et 50 V, pendant un temps de 1 à 10000 s, et notamment entre 5 et 100 s.

Dans une 2^{nde} variante et à titre d'exemple, le procédé préventif selon l'invention peut être mis en oeuvre à l'aide d'un champ électrique variable auquel un matériau composite comprenant des nanotubes de carbone est soumis en appliquant une tension pulsée, comprise entre 50 et 1000 V et, notamment entre 100 et 500 V, avec des impulsions dont la durée est comprise entre 0,1 µs et 100 ms et, notamment, entre 0,1 et 50 ms et dont la fréquence est comprise entre 10 Hz à 100 kHz et, notamment entre 50 Hz et 1000 Hz.

Dans le cadre de la présente invention, par « réduire ou éliminer l'encrassement », on entend un procédé curatif, appliqué à la membrane poreuse à base de nanotubes qui permet d'obtenir une membrane présentant un encrassement moins important que celui obtenu avec la même membrane (i.e. avec un matériau composite identique) utilisée dans les mêmes conditions et pendant le même temps. Le procédé selon l'invention est, dans ce cas, un procédé de traitement d'un matériau composite ou d'une membrane en cours d'utilisation.

Dans une 1^{ère} variante et à titre d'exemple, le procédé curatif peut être mis en oeuvre à l'aide d'un champ électrique constant auquel un matériau composite comprenant des nanotubes de carbone est soumis en appliquant une tension constante, comprise entre 0,1 et 100 V et, notamment entre 1 et 50 V, pendant un temps de 1 à 10000 s, et notamment entre 10 et 8000 s.

Dans une 2^{nde} variante et à titre d'exemple, le procédé curatif peut être mis en oeuvre à l'aide d'un champ électrique constant auquel un matériau composite comprenant des nanotubes de carbone est soumis en appliquant une tension pulsée, comprise entre 50 et 1000 V et, notamment entre 200 et 900 V, avec des impulsions dont la durée est comprise entre 0,1 µs et 100 ms et, notamment entre 0,1 et 90 ms et dont la fréquence est comprise entre 10 Hz à 100 kHz et, notamment entre 50 et 1000 Hz.

Le procédé de désencrassement peut être généré régulièrement tout au long de la vie du matériau composite, soit par prévention, soit par curation.

Il convient de souligner qu'il est possible de suivre le niveau d'encrassement d'une membrane par mesure de la résistance et de déclencher une stratégie de désencrassement adaptée. Dans ce cas, on peut parler de « membrane active ».

Le procédé de désencrassement concerne également un procédé permettant de nettoyer la surface d'un revêtement (ou coating) hydrophobe à base de nanotubes de carbone tel que précédemment défini, présentant des propriétés de super fluidité lorsqu'un liquide, par exemple de l'eau, glisse sur sa surface. En effet, dans le cas d'un revêtement qui serait soumis à un environnement tel qu'un encrassement puisse venir détériorer ses qualités de super fluidité, par exemple un revêtement anti-fouling marin constamment au contact d'eau de mer, les deux stratégies de nettoyage i.e. de désencrassement présentées ci-dessus sont aussi appliquables.

De plus, les nanotubes sont aussi connus pour leur capacité à renforcer un matériau composite, en particulier, un matériau composite piézo-électrique. L'utilisation de nanotubes dans le cas où la matrice est un matériau composite piézo-électrique permet d'améliorer le comportement piézo-électrique du matériau de base **[21]** et donc de mettre en oeuvre des techniques classiques de décolmatage par effet de vibration piézo-électrique.

La présente divulgation décrit également un procédé pour modifier un matériau composite tel que précédemment défini, comprenant une étape consistant à soumettre ledit matériau composite à un champ électrique. Un tel procédé met en oeuvre le dispositif tel que précédemment défini.

Par « pour modifier un matériau composite », on entend un procédé (1) pour séparer en au moins deux parties distinctes un tel matériau composite, (2) pour réduire ou amincir un tel matériau composite, (3) pour augmenter la qualité structurale d'un tel matériau composite et/ou (4) pour créer une matrice mésoporeuse sur un tel matériau composite.

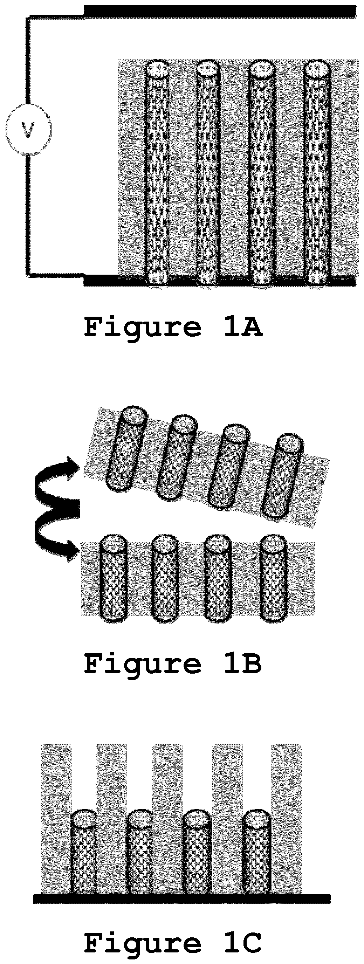

L'effet de champ précédemment décrit peut être utilisé pour séparer en au moins deux parties distinctes un matériau composite tel que précédemment défini i.e. pour découper, dans le sens de son épaisseur, ce matériau composite. La Figure 1A est une représentation de cette découpe. Pour une telle utilisation et à titre d'exemple, le champ électrique auquel un matériau composite comprenant des nanotubes de carbone est soumis, est obtenu en appliquant une tension pulsée, supérieure à 1 kV et, notamment comprise entre 2 et 100 kV, avec des impulsions dont la durée est comprise entre 0,1 µs et 100 ms et, notamment entre 0,1 et 90 ms et dont la fréquence est comprise entre 10 Hz à 100 kHz et, notamment entre 50 et 1000 Hz.

L'effet de champ précédemment décrit peut aussi être utilisé pour réduire l'épaisseur du matériau composite et, plus particulièrement, pour réduire la longueur des nanotubes dans le matériau composite, comme représenté à la Figure 1B. Pour une telle utilisation et à titre d'exemple, le champ électrique auquel un matériau composite selon l'invention comprenant des nanotubes de carbone est soumis est obtenu en appliquant une tension pulsée, supérieure à 1 kV et, notamment comprise entre 2 et 100 kV, avec des impulsions dont la durée est comprise entre 0,1 µs et 100 ms et, notamment entre 0,1 et 90 ms et dont la fréquence est comprise entre 10 Hz à 100 kHz et, notamment entre 50 et 1000 Hz.

L'augmentation de la qualité structurale du matériau composite et notamment des nanotubes traduit l'absence de défauts dans la structure cristalline des nanotubes, la continuité des parois, l'alignement et la rectitude des nanotubes. Elle peut se déterminer par des analyses RAMAN en particulier pour les NTC en regardant les modes RBS et le ratio des modes ID/IG qui doit être le plus proche possible de l'unité. A titre d'exemple, une telle augmentation est mise en oeuvre en soumettant un matériau composite selon l'invention comprenant des nanotubes de carbone à un champ électrique obtenu en appliquant une tension pulsée, supérieure à 1 kV et, notamment comprise entre 2 et 100 kV, avec des impulsions dont la durée est comprise entre 0,1 µs et 100 ms et, notamment entre 0,1 et 90 ms et dont la fréquence est comprise entre 10 Hz à 100 kHz et, notamment entre 50 et 1000 Hz.

Une application du dispositif tel que précédemment défini liée à la modification d'un matériau composite tel que précédemment défini concerne la préparation d'une membrane bi-couche avec une 1^{ère} couche correspondant audit matériau composite et une 2^{nde} couche disposée sur la 1^{ère} correspondant à une membrane microporeuse. Cette application met en oeuvre un matériau composite tel que précédemment « enterré » dans une matrice de remplissage, avantageusement une matrice polymère, qui peut être identique à ou différente de la matrice de remplissage qui le constitue. En effet, l'application d'une tension sur une membrane dont les nanotubes sont enterrés a pour effet de créer des porosités au droit des nanotubes. La porosité ainsi obtenue étant supérieure à celle des nanotubes, la structure réalisée correspond à la superposition d'une membrane microporeuse (matrice seule et notamment polymère seul) sur une membrane nanoporeuse (tapis de nanotubes alignés, fonctionnalisés et imprégnés de matrice de remplissage). La Figure 1C est une schématisation de cette application. Pour une telle utilisation et à titre d'exemple, le champ électrique auquel la membrane dont les nanotubes de carbone sont enterrés dans une matrice est soumise, est obtenu en appliquant une tension pulsée, supérieure à 1 kV et, notamment comprise entre 2 et 100 kV, avec des impulsions dont la durée est comprise entre 0,1 µs et 100 ms et, notamment entre 0,1 et 90 ms et dont la fréquence est comprise entre 10 Hz à 100 kHz et, notamment entre 50 et 1000 Hz.

La présente divulgation décrit enfin un procédé pour électroporer au moins une cellule comprenant les étapes consistant à :
α) mettre ladite cellule en présence d'un matériau composite tel que précédemment défini ;
β) soumettre ledit matériau composite à un champ électrique. Un tel procédé met en oeuvre le dispositif selon l'invention tel que précédemment défini.

Par « électroporer une cellule », on entend un procédé entraînant des dommages au niveau de la membrane plasmique, de la membrane cellulaire, de la capsule et/ou de la paroi cellulaire de la cellule en ouvrant des pores dans ces dernières et ainsi en perméabilisant la cellule. Ces dommages peuvent être réversibles ou irréversibles.

Lorsqu'ils sont réversibles, les dommages causés au niveau de la membrane plasmique, de la membrane cellulaire, de la capsule et/ou de la paroi cellulaire n'affectent que peu la cellule, se présentent comme une poration cellulaire temporaire et peuvent être « réparés » en utilisant la machinerie cellulaire.

Dans ce cas, le procédé pour électroporer au moins une cellule peut être utilisé pour modifier cette dernière. Ainsi, le procédé pour électroporer comprend une étape supplémentaire consistant à modifier la cellule électroporée.

Cette étape additionnelle comprend la mise en contact de la cellule électroporée avec un élément apte à la modifier. Elle peut consister à mettre la cellule à électroporer dans un milieu comprenant un élément apte à la modifier et un matériau composite tel que précédemment défini et ce, préalablement à l'étape (β). En variante, elle peut consister à mettre la cellule déjà électroporée en présence d'un élément apte à la modifier. Par « élément apte à la modifier », on entend une molécule naturelle ou synthétique, notamment une molécule biologique ou biologiquement active, qui peut être apte à transformer la cellule et/ou à en modifier les propriétés, les caractéristiques et/ou le comportement.

Cette étape additionnelle est une technique « classique » d'électroporation. Elle est donc aussi utilisable dans le procédé pour électroporer une cellule décrit dans la présente dont le bénéfice est essentiellement de permettre de générer des champs équivalents aux électrodes classiques mais avec des niveaux de tension plus faibles, donc plus faciles à mettre en oeuvre électroniquement, moins chers et plus économiques énergétiquement.

Dans le cas de dommages réversibles et à titre d'exemple, le champ électrique auquel un matériau composite comprenant des nanotubes de carbone dans une matrice lors de l'étape (β) est soumis, est obtenu en appliquant une tension pulsée comprise entre 1 V et 1 kV et, notamment comprise entre 10 et 100 V, avec des impulsions dont la durée est comprise entre 0,1 µs et 100 ms et, notamment entre 0,1 et 90 ms et dont la fréquence est comprise entre 10 Hz à 100 kHz et, notamment entre 50 et 1000 Hz.

Lorsque les dommages causés sont irréversibles, ils peuvent aller jusqu'à la destruction de la membrane plasmique, de la membrane cellulaire, de la capsule et/ou de la paroi cellulaire et donc à l'éclatement de la cellule. Le procédé pour électroporer une cellule peut donc être mis en oeuvre pour éliminer ou détruire ladite cellule. De fait, il trouve de nombreuses applications dans le traitement et la désactivation des déchets biologiques, le traitement et la conservation des denrées alimentaires telles que l'eau minérale, les jus de fruits, le lait, les boissons lactées et autres et/ou dans le traitement thérapeutique.

Dans le cas de dommages irréversibles et à titre d'exemple, le champ électrique auquel un matériau composite comprenant des nanotubes de carbone dans une matrice lors de l'étape (β) est soumis, est obtenu en appliquant une tension pulsée, comprise entre 1 V et 1 kV et, notamment comprise entre 10 et 100 V, avec des impulsions dont la durée est comprise entre 0,1 µs et 100 ms et, notamment entre 0,1 et 90 ms et dont la fréquence est comprise entre 10 Hz à 100 kHz et, notamment entre 50 et 1000 Hz.

On entend par « cellule », aussi bien une cellule de type procaryote que de type eucaryote. Parmi les cellules eucaryotes, la cellule peut être une levure telle qu'une levure du genre *Saccharomyces* ou *Candida,* une cellule animale et notamment une cellule de mammifères, une cellule végétale ou une cellule d'insectes. Les cellules de mammifères peuvent notamment être des cellules tumorales, des cellules de lignée somatique normale ou des cellules souches. Les cellules de type procaryote sont des bactéries qui peuvent être des gram + ou -. Parmi ces bactéries, on peut citer, à titre d'exemples et de façon non-exhaustive, les bactéries appartenant aux embranchements des spirochètes et des chlamydiae, les bactéries appartenant aux familles des entérobactéries (telles que *Escherichia coli*), des streptococcacées (telles que streptococcus), des microccacées (telles que staphylococcus), des légionelles, des mycobactéries, des bacillacées et autres.

Les cellules mises en oeuvre peuvent se présenter sous forme d'une culture cellulaire en suspension ; sous forme d'une culture cellulaire attachée sur un support tel qu'une boîte de culture ou une boîte multipuits ; sous forme d'un tissu « ex vivo » ; sous forme d'un tissu « in vivo » ; dans un échantillon liquide tel qu'une denrée alimentaire ou un déchet biologique ; dans un fluide biologique préalablement extrait d'un corps humain ou animal, ledit échantillon pouvant avoir subi différents traitements préalables comme une centrifugation, une concentration, une dilution ....

Il est clair que le procédé pour électroporer au moins une cellule peut être mis en oeuvre aussi bien *in vivo* qu'*in vitro.* Il peut donc être comparé dans certains cas et notamment lorsque des molécules à visée thérapeutique sont introduites dans la (ou les) cellule(s) électroporé(es) à un traitement thérapeutique.

Dans le cadre du procédé d'électroporation décrit dans la présente, le matériau composite et la contre-électrode peuvent être disposés dans les milieux liquides précédemment envisagés dans lesquels se trouve(nt) la (ou les) cellule(s) à électroporer ; au niveau des récipients contenant lesdits milieux liquides ; sur les supports sur lesquels se trouve(nt) la (ou les) cellule(s) à électroporer ; dans les tissus où se trouve(nt) la (ou les) cellule(s) à électroporer.

Dans la présente description, des valeurs avantageuses quant à la tension, la fréquence et la durée des impulsions sont données pour des cas de figures particuliers. Sur la base de cet enseignement, l'homme du métier saura déterminer, pour un cas de figure différent, les valeurs optimales à utiliser en tenant compte des paramètres d'influence qui sont :
- les paramètres de forme de l'électrode ou de la membrane : longueur des nanotubes, densité (i.e. distance entre chacun), diamètre, nombre de parois pour les NTCs, qualité d'alignement, résistance (du nanotube et transverse à la membrane i.e. nombre de connexion qui vont réduire l'effet de champ) ;
- les paramètres classiques de l'électroporation : type de liquide (essentiellement sa conductivité électrique, sa turbidité et la mobilité des espèces), le type et la taille de la cellule à électroporée et le type de membrane cellulaire ;
- les paramètres environnementaux : température, pression, écoulement etc ...

L'invention sera mieux comprise à la lecture des figures et exemples qui suivent. Ceux-ci n'ont pas pour but de limiter l'invention dans ses applications, il ne s'agit que d'illustrer ici les possibilités offertes par le procédé de l'invention.

### BRÈVE DESCRIPTION DES DESSINS

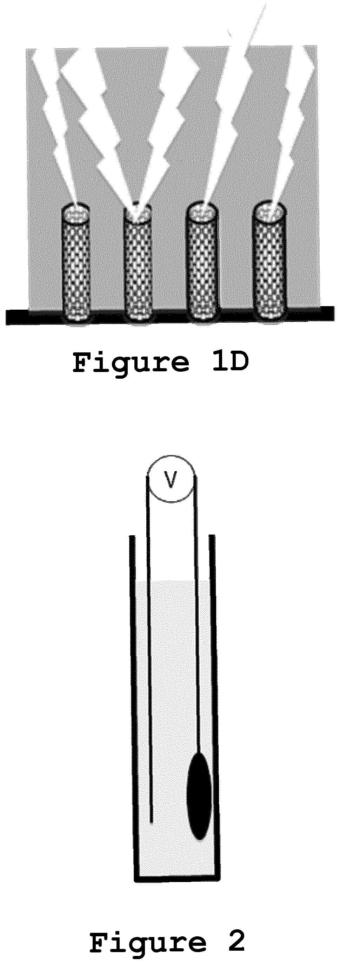

La Figure 1 ne faisant pas partie de l'invention revendiquée présente une schématisation de différentes applications de l'effet de champ pour modifier un matériau composite comprenant un tapis de nanotubes imprégné dans une matrice de remplissage et soumis à une forte tension (Figure 1A). Les différentes applications schématisées sont la découpe d'un tel matériau composite (Figure 1B), l'amincissement d'un tel matériau composite par raccourcissement des tapis de nanotubes dans la matrice (Figure 1C) et la réalisation d'une membrane comprenant un tel matériau composite sur lequel une membrane microporeuse est disposée avec création de chemin de percolation au droit des tapis dans la matrice (Figure 1D).
La Figure 2 présente une schématisation d'un dispositif mis en oeuvre dans la présente invention.
La Figure 3 présente une image en microscopie électronique à balayage d'une membrane encrassée (Figure 3A) et d'une membrane désencrassée suite à la mise en oeuvre d'un procédé selon l'invention (Figure 3B).

### EXPOSÉ DÉTAILLÉ DE MODES DE RÉALISATION PARTICULIERS

### I. Préparation du matériau composite utilisable dans le dispositif selon l'invention.

### I.1. Tapis de nanotubes de carbone.

La croissance des tapis de nanotubes de carbone est réalisée par la technique « Aerosol assisted CVD » sur quartz (mélange ferrocène/toluène à 2,5%, Argon comme gaz porteur).

La dimension du tapis est de 800 µm, les nanotubes ont un diamètre externe moyen de 40 nm et interne moyen de 8 nm. La taille du tapis ainsi obtenu est de 1,5 cm x 1,5 cm.

Le substrat est retiré en un seul morceau en utilisant une lame de rasoir.

### 1.2. Réalisation d'un matériau composite.

Dans un pot, quelques gouttes d'une solution mère de résine époxy sont déposées sur le tapis seul. La solution mère de résine époxy est composée des éléments suivants :
- 7,5 ml d'EPON (pour « Epoxy Embedding Medium, Glycerol Glycidyl Ether »);
- 6,5 ml de MNA (pour « Methylnadic anhydride ») ;
- 0,110 ml de DMP30 (2,4,6-tris(dimethylaminomethyl)phenol).

L'échantillon est placé dans une étuve à 60°C. Pendant 30 min, un vide primaire est maintenu dans l'étuve puis l'échantillon est laissé 16 h à cette température. Ensuite, on coupe la température pour revenir à la température normale en 12 h.

L'échantillon est aminci par polissage mécanique de disques abrasifs de rugosité décroissante (taille de grains par exemple P120, P180, P320, P600 pour amincir). L'amincissement est réalisé successivement sur les deux faces. Puis des disques abrasifs de taille de grains, par exemple, P1200, P2400 et P4000 sont successivement utilisés sur les deux faces et ce, pour polir. L'épaisseur obtenue de la membrane est voisine de 100 µm.

L'échantillon est nettoyé à l'eau puis à l'éthanol pour retirer les résidus du polissage.

Enfin, une couche conductrice d'or a été déposée, à l'aide d'un évaporateur sous vide, sur un seul côté de la membrane. L'épaisseur de la couche est de l'ordre de 100 nm. Elle n'a qu'un rôle d'amélioration de la conduction électrique.

Trois membranes identiques ci-après désignées Mem1, Mem2 et Mem3 sont préparées comme indiqué ci-dessus. La membrane Mem1 est gardée intacte comme référence.

Les membranes Mem2 et Mem3 sont plongées dans une solution saline réelle (eau saumâtre de l'étang de Berre) pendant 6 mois afin que les algues et le biofilm puissent se développer. Les conditions d'aérobie ne sont pas contrôlées, seul le bon développement du biofilm est contrôlé.

### II. Dispositif et utilisation.

Le dispositif mis en oeuvre est présenté Figure 2.

Il comprend :
- la membrane Mem3 à NTCs alignés et matrice époxy préparée au point I après son séjour dans la solution saline réelle (Mem3) ;
- une contre-électrode sous forme d'un fil de cuivre (CE) ;
- une alimentation en tension pulsée (V).

Plus particulièrement, la membrane Mem3 est accrochée à un fil électrique par une pince. Un fil en Cuivre est apporté comme contre électrode. On s'assure que, pendant toute la durée de l'expérience, il n'y a pas de contact entre les deux.

On utilise un générateur de courant capable de produire une tension de 100 V à des impulsions de l'ordre de la microseconde. On soumet une séquence de 100 V d'amplitude, 10 µs de durée d'impulsion et de 50 µs de fréquence, pendant 10 min.

Le dispositif est placé dans un électrolyte du type eau de mer.

### III. Résulats.

On sèche les membranes Mem2 après son séjour dans la solution saline réelle du point I et Mem3 suite au protocole décrit au point II dans une étuve sous vide primaire pendant 4 h pour observations au microscope et comparaison avec la membrane Mem1.

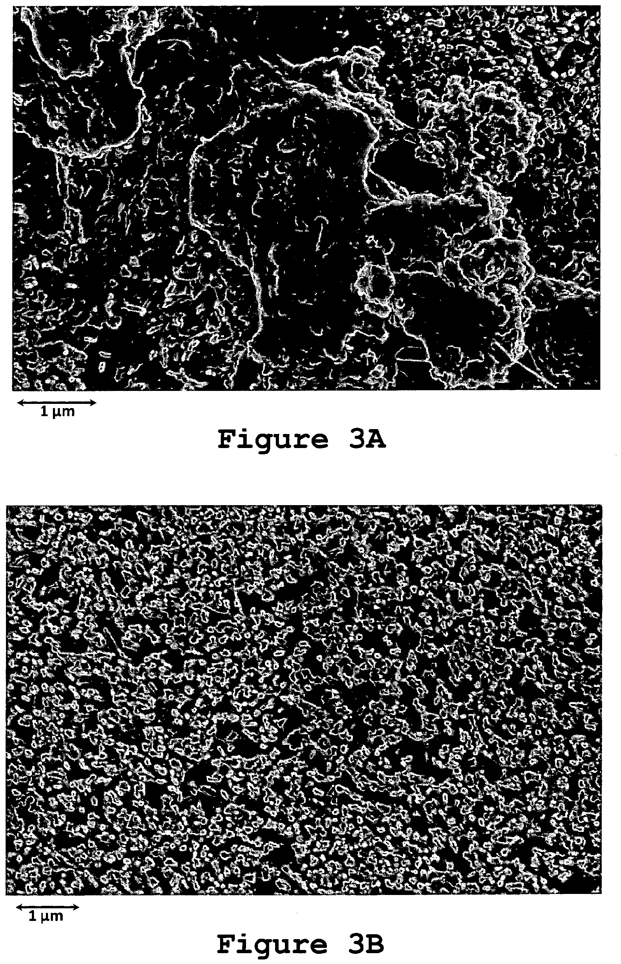

Il apparaît clairement que la membrane Mem3 nettoyée conformément à un procédé de décolmatage selon l'invention présente une surface propre (Figure 3B) Cette surface est proche de celle de la membrane de référence Mem1.

Au contraire, la membrane Mem2 est colmatée suite à son séjour dans la solution saline réelle (Figure 3A).

### RÉFÉRENCES

**[1]** Hummer et al., 2001, « Water conduction through the hydrophobic channel of a carbon nanotube », Nature, vol. 414, pages 188-190.
**[2]** Kang et al., 2007, « Single-walled carbon nanotubes exhibit strong antimicrobial activity », Langmuir, vol. 23, pages 8670-8673.
**[3]** Brady-Estevez et al., 2010, « Multi-walled nanotube filter : improving viral removal at low pressure », Langmuir, vol. 26, pages 14975-14982.
**[4]** Membrane for Water treatment , Ed. K. Peinemann and S. Pereira-Nunes, Wiley-VCH, 2010
**[5]** Demande internationale WO 2011/063458 déposée au nom de University of Sidney et publiée le 3 juin 2011.
**[6]** Cui et al., 2003, « The use of gas bubbling to enhance membrane processes », J. of Membrane Sci., vol. 221, pages 1-35.
**[7]** Bonard et al., 2002, « Field émission of individual carbon nanotubes in the scanning electron microscope », Phys Rev. Lett., vol. 89, pages 197602.
**[8]** Thèse de Marchand, 2009, "Synthèse in-situ et caractérisation de nanotubes de carbone individuels sous émission de champ », Université de Lyon, paragraphes 2.3.1 et 2.3.2, pages 62 à 64.
**[9]** Yantzi et Yeow, 2005, « Carbon nanotubes enhanced pulsed electric field electroporation for médical applications », Mechatronics and automation, 2005 IEEE International Conférence, vol. 4, pages 1872-1877.
**[10]** Demande internationale WO 2008/062378 déposée au nom de Scuola Superiore di Studi Universitari e di Perfezionamento Sant'Anna et publiée le 29 mai 2008.
**[11]** Rojas-Chapana et al., 2005, « Multi-walled carbon nanotubes for plasmid delivery into Escherichia coli cells », Lab. Chip, vol. 5, pages 536-539.
**[12]** Demande internationale WO 2008/028155 déposée au nom de Virginia Tech Intellectual Properties et publiée le 6 mars 2008.
**[13]** Demande de brevet US 2004/0173506 déposée au nom de Doktyez et al. et publiée le 9 septembre 2004.
**[14]** Demande internationale WO 2009/141528 déposée au nom du Commissariat à l'Energie Atomique et publiée le 26 novembre 2009.
**[15]** Pichot et al., 2004, « Evidence of strong nanotube alignment and for iron preferential growth axis in multiwalled carbon nanotube carpets », Applied Physics Letters, vol. 85, pages 473-475.
**[16]** Bahr et al., 2002, « Covalent chemistry of single-wall carbon nanotubes », J. Mater. Chem., vol. 12, pages 1952-1958.
**[17]** Demande internationale WO 2008/078052 déposée au nom du Commissariat à l'Energie Atomique et publiée le 3 juillet 2008.
**[18]** Mévellec et al., 2007, « Grafting polymers on surfaces : a new powerful and versatile diazoniumsalt-based one-step process in aqueous média », Chem. Mater., vol. 19, pages 6323-6330.
**[19]** Tessier et al., 2008, « Grafting organic polymer films on surfaces of carbon nano-tubes by Surface Electroinitiated Emulsion Polymerization », Physica Status Solidi A, vol. 205, pages 1412-1418.
**[20]** Fan et al., 2007, « Synthesis of polymer grafted carbon nanotubes by nitroxide mediated radical polymerization in the presence of spin*-labeled carbon nanotubes », Macromolecular Nanotechnology, vol. 43, pages 26-34.
**[21]** Tian et Wang, 2008, « Fabrication and performances of epoxy/multi-walled carbon nanotubes/piezoelectric ceramic composites as rigid piezo-damping material », J. Mater. Sci., vol. 43, pages 4979-4987.

## Revendications

1. Utilisation d'un dispositif comprenant :
i) un matériau composite comprenant (1) une pluralité de nanotubes conducteurs ou semi-conducteurs alignés les uns par rapport aux autres, et (2) une matrice disposée entre ces nanotubes, ledit matériau composite se présentant sous forme d'une membrane isoporeuse dont les nanopores correspondent aux pores des nanotubes et éventuellement aux pores de la matrice de remplissage ; et
ii) des moyens permettant de soumettre ledit matériau composite à un champ électrique,
pour empêcher, réduire et/ou éliminer l'encrassement à la surface, au niveau et dans les pores dudit matériau composite.

2. Utilisation d'un dispositif selon la revendication 1, **caractérisée en ce que** tout ou partie des nanotubes conducteurs ou semi-conducteurs sont des nanotubes inorganiques, avantageusement choisis dans le groupe constitué par des nanotubes d'oxyde de zinc (ZnO), des nanotubes de nitrure de gallium (GaN), de nitrure de Bore (BN), des nanotubes du bisulfure de tungstène (WS₂), des nanotubes de bisulfure de molybdène (MoS₂), des nanotubes de séléniure de tungstène (WSe₂), des nanotubes de dioxyde de titane (TiO₂) ou des nanotubes de trioxyde de molybdène (MoO₃) ou un de leurs mélanges.

3. Utilisation d'un dispositif selon la revendication 1 ou 2, **caractérisée en ce que** tout ou partie des nanotubes conducteurs ou semi-conducteurs sont des nanotubes organiques et avantageusement des nanotubes de carbone.

4. Utilisation d'un dispositif selon l'une quelconque des revendications précédentes, **caractérisée en ce que** lesdits nanotubes se présentent sous forme d'un tapis de nanotubes.

5. Utilisation d'un dispositif selon l'une quelconque des revendications précédentes, **caractérisée en ce que** la densité desdits nanotubes est comprise entre 10⁴ et 10¹³ nanotubes/cm² de matériau composite, avantageusement de l'ordre de 10⁵ à 10¹³ nanotubes/cm² et notamment de l'ordre de 10⁹ à 10¹¹ nanotubes/cm².

6. Utilisation d'un dispositif selon l'une quelconque des revendications précédentes, **caractérisée en ce qu'**au moins un polymère organique est greffé sur les nanotubes conducteurs ou semiconducteurs.

7. Utilisation d'un dispositif selon l'une quelconque des revendications précédentes, **caractérisée en ce que** ladite matrice est choisie dans le groupe constitué par une matrice céramique, une matrice polymère, une matrice métallique, une matrice issue de la biomasse ou une matrice issue de dérivés cellulosiques et leurs mélanges.

8. Utilisation d'un dispositif selon l'une quelconque des revendications précédentes, **caractérisée en ce que** lesdits moyens sont choisis parmi des contacts électriques, une ou plusieurs électrode(s) et une source de tension.

9. Utilisation d'un dispositif selon l'une quelconque des revendications précédentes, **caractérisée en ce que** lesdits moyens sont au moins une électrode et au moins une source de tension électriquement reliée, d'une part, au matériau composite tel que défini à l'une quelconque des revendications 1 à 7 et, d'autre part, à ladite au moins une électrode.

10. Utilisation d'un dispositif selon l'une quelconque des revendications précédentes, **caractérisée en ce que** ledit champ électrique est continu ou variable.

## Patentansprüche

1. Verwendung einer Vorrichtung, umfassend:
i) ein Verbundmaterial, umfassend (1) eine Mehrzahl von leitenden oder halbleitenden Nanoröhrchen, die zueinander ausgerichtet sind, und (2) eine Matrix, die zwischen diesen Nanoröhrchen angeordnet ist, wobei das Verbundmaterial die Form einer isoporösen Membran aufweist, deren Nanoporen den Poren der Nanoröhrchen und gegebenenfalls den Poren der Füllmatrix entsprechen; und
ii) Mittel, die es ermöglichen, das Verbundmaterial einem elektrischen Feld auszusetzen,
zum Verhindern, Verringern und/oder Beseitigen einer Verschmutzung des Verbundmaterials an der Oberfläche im Bereich der und in den Poren.

2. Verwendung einer Vorrichtung nach Anspruch 1, **dadurch gekennzeichnet, dass** alle oder ein Teil der leitenden oder halbleitenden Nanoröhrchen anorganische Nanoröhrchen sind, vorzugsweise ausgewählt aus der Gruppe gebildet durch Nanoröhrchen aus Zinkoxid (ZnO), Nanoröhrchen aus Galliumnitrid (GaN), aus Bornitrid (BN), Nanoröhrchen aus Wolframbisulfid (WS₂), Nanoröhrchen aus Molybdenbisulfid (MoS₂), Nanoröhrchen aus Wolframselenid (WSe₂), Nanoröhrchen aus Titandioxid (TiO₂) oder Nanoröhrchen aus Molybdentrioxid (MoO₃), oder eine ihrer Mischungen.

3. Verwendung einer Vorrichtung nach Anspruch 1 oder 2, **dadurch gekennzeichnet, dass** alle oder ein Teil der leitenden oder halbleitenden Nanoröhrchen organische Nanoröhrchen sind, und vorzugsweise Kohlenstoff-Nanoröhrchen.

4. Verwendung einer Vorrichtung nach einem der vorhergehenden Ansprüche, **dadurch gekennzeichnet, dass** die Nanoröhrchen die Form eines Bands von Nanoröhrchen aufweisen.

5. Verwendung einer Vorrichtung nach einem der vorhergehenden Ansprüche, **dadurch gekennzeichnet, dass** die Dichte der Nanoröhrchen enthalten ist zwischen 10⁴ und 10¹³ Nanoröhrchen/cm² von Verbundmaterial, vorzugsweise in der Größenordnung von 10⁵ bis 10¹³ Nanoröhrchen/cm² und insbesondere in der Größenordnung von 10⁹ bis 10¹¹ Nanoröhrchen/cm².

6. Verwendung einer Vorrichtung nach einem der vorhergehenden Ansprüche, **dadurch gekennzeichnet, dass** wenigstens ein organisches Polymer auf die leitenden oder halbleitenden Nanoröhrchen gepfropft ist.

7. Verwendung einer Vorrichtung nach einem der vorhergehenden Ansprüche, **dadurch gekennzeichnet, dass** die Matrix ausgewählt ist aus der Gruppe gebildet durch eine keramische Matrix, eine Polymermatrix, eine Metallmatrix, eine aus Biomasse erzeugte Matrix oder eine aus Zellulosederivaten erzeugte Matrix und ihre Mischungen.

8. Verwendung einer Vorrichtung nach einem der vorhergehenden Ansprüche, **dadurch gekennzeichnet, dass** die Mittel ausgewählt sind aus elektrischen Kontakten, einer oder mehreren Elektrode(n) und einer Spannungsquelle.

9. Verwendung einer Vorrichtung nach einem der vorhergehenden Ansprüche, **dadurch gekennzeichnet, dass** die Mittel wenigstens eine Elektrode und wenigstens eine Spannungsquelle sind, die elektrisch einerseits mit dem Verbundmaterial wie in einem der Ansprüche 1 bis 7 definiert verbunden sind, und andererseits mit der wenigstens einen Elektrode.

10. Verwendung einer Vorrichtung nach einem der vorhergehenden Ansprüche, **dadurch gekennzeichnet, dass** das elektrische Feld konstant oder variabel ist.

## Claims

1. Use of a device comprising:
i) a composite material comprising (1) a plurality of conductive or semiconductive nanotubes aligned relative to one another, and (2) a matrix arranged between these nanotubes; said composite material being in the form of an isoporous membrane whose nanopores correspond to the pores of the nanotubes, and optionally to the pores of the filler matrix; and
ii) means allowing said composite material to be subjected to an electric field,
to prevent, reduce and/or eliminate the fouling on the surface, at and in the pores of said composite material.

2. The use of a device according to claim 1, **characterized in that** all or part of the conductive or semiconductive nanotubes are inorganic nanotubes advantageously chosen from the group consisting of nanotubes of zinc oxide (ZnO), nanotubes of gallium nitride (GaN), of boron nitride (BN), nanotubes of tungsten bisulfide (WS₂), nanotubes of molybdenum bisulfide (MoS₂), nanotubes of tungsten diselenide (WSe₂), nanotubes of titanium dioxide (TiO₂) or nanotubes of molybdenum trioxide (MoO₃), or a mixture thereof.

3. The use of a device according to claim 1 ou 2, **characterized in that** all or part of the conductive or semiconductive nanotubes are organic nanotubes and advantageously carbon nanotubes.

4. The use of a device according to any of the preceding claims, **characterized in that** said nanotubes are in the form of a nanotube carpet.

5. The use of a device according to any of the preceding claims, **characterized in that** the density of the said nanotubes is between 10⁴ and 10¹³ nanotubes/cm² of composite material, advantageously in the order of 10⁵ to 10¹³ nanotubes/cm² and in particular in the order of 10⁹ a 10¹¹ nanotubes/cm².

6. The use of a device according to any of the preceding claims, **characterized in that** at least one organic polymer is grafted onto the conductive or semiconductive nanotubes.

7. The use of a device according to any of the preceding claims, **characterized in that** said matrix is chosen from the group consisting of a ceramic matrix, polymer matrix, metal matrix, a matrix derived from biomass or a matrix derived from cellulose derivatives and mixtures thereof.

8. The use of a device according to any of the preceding claims, **characterized in that** said means are chosen from among electrical contacts, one or more electrodes and a voltage source.

9. The use of a device according to any of the preceding claims, **characterized in that** said means are at least one electrode and at least one voltage source that is connected both to the composite material such as defined in any of claims 1 to 7 and to said at least one electrode.

10. The use of a device according to any of the preceding claims, **characterized in that** said electric field is continuous or variable.
